# EUROPEAN PATENT SPECIFICATION

(11) **EP 1 587 453 B1**
(45) Date of publication and mention of the grant of the patent: **17.09.2008**
(21) Application number: 04707465.3
(22) Date of filing: 02.02.2004
(51) Int. Cl.: A61F 2/06, A61L 31/06

(54) **ABSORBABLE / BIODEGRADABLE TUBULAR STENT AND METHODS OF MAKING THE SAME**
RESORBIERBARER / BIOLOGISCH ABBAUBARER RÖHRENFÖRMIGER STENT UND HERSTELLUNGSVERFAHREN DAFÜR
STENT TUBULAIRE ABSORBABLE/BIODEGRADABLE ET SON PROCEDE DE FABRICATION

(30) Priority: 31.01.2003 US 444023 P; 31.01.2004 US 768834
(43) Date of publication of application: 26.10.2005
(73) Proprietor: Poly-Med, Inc., Anderson, SC 29625 (US)
(72) Inventor: SHALABY, Shalaby, W., Anderson, SC 29625 (US)
(74) Representative: Wüstefeld, Regine Marie
(86) International application number: PCT/US2004/003192
(87) International publication number: WO 2004/069097

(56) References cited:
- WO-A2-01/40348
- WO-A2-95/26695
- US-A- 5 593 434
- US-A1- 2002 114 840
- US-B1- 6 197 320
- US-B1- 6 679 910
- US-B2- 6 626 936
- US-B2- 6 645 243

## Description

### Field of the Invention

This invention relates to an absorbable/biodegradable, radially fluted, tubular stent having grooves or flutes along its entire length for expansion to a predetermined range of diameters, depending on the number and variability in the shape and depth of the flutes or grooves, after deployment, using a balloon-catheter, in a tubular body lumen through outward deformation of said grooves to yield an essentially circular cross-section to stabilize the internal dimensions of the treated conduit or lumen as in the case of an endovascular stent that is used in preventing vascular restenosis.

### Background of the Invention

Stents, including cardiovascular and biliary stents, are well known as devices that are used to support a body lumen, such as an artery, vein, biliary duct, or esophagus. They may be employed as a primary treatment for a construction of a body lumen (stenosis), or may be used following a medical procedure, such as angioplasty, used to remedy stenosis.

Conventional stents have taken two forms. First, there are the self-expanding stents that typically are made of metal and that may include a biocompatible coating. Such stents are permanently implanted into the human body by deploying them on or through a catheter, although removable stents of this kind are known to the art. The stent, which may be woven, strutted, or wound like a spring, is placed in tension or compression along the inner or outer perimeter of the catheter, and percutaneously inserted into the body where it is guided to the site of implantation. The stent then is released from the perimeter of the catheter, or extruded from the interior of the catheter, where it expands to a fixed, predetermined diameter, and is held in position as a result of that expansion. Many different configurations of such self-expanding stents, and of catheters used to deploy such stents, are known to the art.

One variation on these self-expanding stents is illustrated in Kawai et al., U.S. Pat. No. 4,950,258. Kawai discloses the use of a spring-like coil of plastic having "shape memory." The stent is manufactured to a desired size from homopolymers or copolymers of lactide and/or glycolide, and then compressed under suitable conditions for insertion into the body. Thereafter, the stent is heated, and because of "shape memory," returns to its original (uncompressed) size.

A second type of stent commonly used in the field is expandable as a result of mechanical action by the surgeon. One such stent is disclosed in Palmaz, U.S. Pat. Nos. 4,733,665, 4,776,337, and 4,639,632. According to the Palmaz patents, an unexpanded stent is permanently implanted in the body by percutaneously inserting it into a vessel using a catheter, and guiding the stent to the site where it is to be permanently implanted. Upon reaching the site of implantation, the balloon portion of the catheter is expanded and concomitantly a portion of the stent also is expanded solely as a result of the mechanical force applied by the expanding balloon, until the stent is sized appropriately for the implantation site. Thereafter, the expanded balloon is deflated, and the catheter is removed from the body, leaving the stent held permanently in position. The stents disclosed in Palmaz are made of a metal or a nondegradable plastic and, to achieve compatibility with and in the body, the stent may be coated with a biologically compatible substance.

Commercially available stents of the types described above exhibit undesirable characteristics that the art has sought to overcome. Self-expanding stents may be inappropriately sized for the sites where they are to be deployed, increasing the risk of rupture, stent migration, stenosis, and thrombosis as the stent continually tries to expand after deployment to its predetermined, optimal diameter. Conversely, a stent sized too small for the lumen may project into the lumen, thereby causing a primary or secondary obstruction or migration. Both self-expanding and expandable stents that are know in the art, because they are designed for permanent implantation in the body, increase the risk of restenosis, thrombosis, or other adverse medical effects because of the risk of adverse reaction by surrounding tissue, adverse reaction by the material flowing through the body lumen (such as blood or blood products), and deterioration of surrounding tissue and/or the stent itself. The metals or alloys used for such stents, because they are believed to be biologically stable, also remain in the body for the patient's life, unless surgically removed at a later date along with surrounding tissue. Thus, these stents do not permit temporary placement within the body unless patient and surgeon are prepared to undertake a second procedure to remove the stent, which is difficult or impossible in most cases.

Conventional balloon-deployed stents, like that described by Palmaz, also require an extensively perforated structure that can be mechanically expanded intraluminally by a balloon catheter without applying forces that are potentially threatening to the surrounding tissue. Such perforations also permit cell growth to occur from the intima or media lining the lumen. Thus, for example, endothelial cells and smooth muscle fibroblasts migrate through the perforations inside and around stents like that shown in Palmaz. Such endothelial cell growth is desirable to the extent that the endothelial layer inhibits the formation of blood clots (thrombogenesis) by providing a blood-compatible surface. However, vascular smooth muscle cell migration and proliferation may be undesirable when it is uncontrolled (as in intimal hyperplasia) and results in the occlusion of the lumen that has been surgically opened by placement of the stent. Thus, stents such as that described by Palmaz may be undesirable when the risk of intimal hyperplasia is substantial. The benefits of a balloon-deployed stent, therefore, may not be realized in such circumstances. Moreover, to the extent that the design of stents such as those described in Palmaz are dictated primarily by mechanical considerations, such as the forces needed to open the stent, biological considerations (such as designing the stent to limit cell ingrowth and migration, for example) frequently play a secondary role or no role at all.

Still another disadvantage of existing stents is that the materials from which they are made are rigid, and therefore, the compliance of the stents (i.e., the ability to control the flexibility of the material used to design stents for particular applications) is limited. This has the disadvantage of exposing patients to risks associated with the placement of a device that may exhibit a rigidity in excess of that needed for the particular application.

Most conventional stents also are capable of being used as drug delivery systems when they are coated with a biodegradable coating that contains the drug to be delivered. The amount of the drug that can be delivered, and the time over which it may be released, therefore, may be limited by the quality of coating employed.

Beck et al., U.S. Pat. No. 5,147,385, discloses the use of a degradable, mechanically expandable stent prepared from poly(ε-caprolactone) or similar polymers that melt between 45°-75°C, because the melted polymer may be expanded in such a manner as to adapt to the body lumen in which it is deployed. At the same time, because poly(ε-caprolactone) enters a liquid phase in the temperature range that Beck discloses (at about 60°C), the ability to achieve controlled, improved strength characteristics using the stent described by Beck is limited. Furthermore, the temperature range described by Beck et al. is well above the glass-transition temperature of poly(ε-caprolactone). This limits the ability of a stent made according to Beck et al. to resist radially compressive forces imparted by the lumen upon the stent without creeping or relaxing, introducing a substantial risk of occluding the lumen. Alternatively, one might use massive structures made according to Beck et al. to keep the lumen open, but in so doing, the normal function of the lumen would be perturbed significantly, possibly creating regions where flow of body liquids through the lumen would be severely restricted or stagnate, so that clots may form in those regions.

Slepian et al., U.S. Pat. No. 5,213,580, discloses an endoluminal sealing process using a poly(caprolactone) material that is flowable at temperatures above 60°-80°C. According to Slepian, this flowable material is able to conform to irregularities on the inner surface of the body lumen in which it is deployed.

Goldberg et al., U.S. Pat. No. 5,085,629, discloses the manufacture of a urethral stent made from a terpolymer of *l*-lactide, glycolide, and ε-caprolactone, which is selected to permit the stent to degrade within the body. Goldberg does not, however, disclose the use of an expandable stent, nor does Goldberg et al. provide any information regarding the design of the stent or its method of deployment within the body.

U.S. Pat. No. 6,248,129 B discloses an expandable, biodegradable stent for use within a body lumen comprising a hollow tube made from a copolymer of *l*-lactide and ε-caprolactone that, in expanded form, is of a first diameter sufficient to be retained upon a balloon catheter for placement within the body lumen, and that is not plastically expandable at normal body temperatures, and that is expandable using thermo-mechanical means at a temperature between about 38°-55°C when the balloon catheter is inflated to a second diameter sufficient to be retained within the body lumen. However, it is believed by the present inventor that (1) the temperature required for expansion is close to 55°C and can damage the vital tissue; and (2) the mechanical stability of the expanded configuration would be far less than optimal as one recognizes the stress-relaxation of a device having such shape.

U.S. Pat. No. 5,670,161 discloses a stent comprising a hollow, substantially cylindrical member formed of a biocompatible composition, said composition being in the form of a polymer matrix and at least one medical agent in a weight up to 90 percent of the total weight of the member dispersed uniformly through the polymer matrix, whereby when the stent is disposed in the lumen of the blood vessel, at least one medical agent is released at a controlled release rate from the member into the vessel, it must be dissolved in the polymer matrix and thereafter diffuse through the polymer matrix, and the controlled release rate extending over a period of time after the lumen stent is inserted into the vessel and being controlled solely by the rate of diffusion of the medical agent from the stent. However, it is believed by the present inventor that the force required to expand the stent subject of U.S. Pat. No. 5,670,161 would exceed that usually encountered during angioplasty.

WO 95/26695 discloses a foldable stent or stent graft which may be delivered with or on a catheter or via surgical or other techniques and which is then expanded or unfolded.

Thus, a stent that overcomes the problems just identified, while at the same time providing or enhancing the benefits that result from the use of stents, is needed to improve patient safety and recovery. This provided the incentive to pursue the subject of the present invention.

### Summary of the Invention

The present invention is directed an absorbable, biodegradable, radially fluted, tubular polymeric stent having at least two grooves extending along its entire length for expansion after deployment through outward deformation of the grooves to yield an essentially circular cross-section. In a preferred embodiment the stent has from 3 to 12 grooves extending along the entire length thereof.

Preferably, the stent is formed of an absorbable crystalline polyester having chain sequences derived from at least one cyclic monomer such as *l*-lactide, glycolide, p-dioxanone, trimethylene carbonate, ε-caprolactone, morpholine-2,5-dione. In one embodiment one of the segments/blocks is amorphous and another of the segments/blocks is crystalline. In one embodiment the copolyester has a monocentric polyaxial amorphous core having the crystalline segments/blocks extending outward therefrom.

The stent is formed of a segmented/block copolyester wherein one of the segments/blocks is crystalline and exhibits a melting temperature (Tₘ) of less than about 110°C and another crystalline segment/block exhibits a melting temperature (Tₘ) of from about 140° C to about 220°C. For such embodiment that copolyester may be based on a monocentric polyaxial system having polyaxial core segments with segments/blocks extending outwardly therefrom, wherein the core segments have a lower melting temperature (Tₘ) than the outwardly extending segments/blocks.

In another embodiment the inventive stent is formed of a blend of at least two absorbable polymers which are a dispersed phase of crystalline microrods in an amorphous matrix.

In yet another embodiment the present inventive stent is formed of a blend of at least two absorbable polymers comprising a dispersed phase of crystalline microrods in a matrix, wherein the microrods exhibit a higher degree of crystallinity than the matrix.

In a still further embodiment the present inventive stent is formed of a chitosan-based material, preferably an acylated chitosan, coated with an absorbable polyester.

In one embodiment the outer wall of the unexpanded stent has radially extending barbs to restrict movement of the deployed expanded stent.

Preferably, the present stent is made by a which includes the steps of forming an unfluted tube and thermofonning the grooves therein. The step of forming an unfluted tube may be achieved by a variety of means such melt-extrusion or electrostatic spinning of a viscous solution of the constituent polymer or polymer blend. The latter process step produces a microporous structure. For such embodiment the viscous solution may be formed of chitosan-based materials, and the process may further include the step of coating the formed tube with an absorbable polyester coating. Preferably the tube is acylated prior to coating.

In another preferred embodiment the present inventive stent is made by injection molding.

The present inventive stent is especially suitable for use in vascular and urinogenital applications.

For many applications it is preferred that the stent contains at least one bioactive agent for preventing restenosis and infection. For some applications it is preferred that the stent contains at least 10 percent by weight of an inorganic radiopacifier.

The present invention also is directed to an absorbable, expandable, unfluted, tubular polymeric stent having radially extending barbs. Preferably the wall of the unexpanded form of this stent is perforated. As with the embodiments discussed above, it is preferred that this stent is formed of a segmented/block copolymer.

### Brief Description of the Figures of the Drawing

The accompanying drawings, which are incorporated in and constitute a part of the specification, illustrate presently preferred embodiments of the present invention and, together with the general description given above and the detailed description of the preferred embodiments given below, serve to explain the principles of the present invention.
Figure 1A is a top view of a fluted stent showing the location of three round grooves;
Figure 1B is a side view of the stent of Figure 1A showing that the grooves run the length of the stent.
Figure 2A is a top view of a fluted stent with trapezoidal grooves.
Figure 2B is a side view of the stent of Figure 2A.
Figure 3A is a top view of a fluted stent of the present invention showing the grooves and barbs.
Figure 3B is a side view of the stent of Figure 3A.
Figure 4A is a top view of a circular stent in accordance with the present invention showing the location of the barbs.
Figure 4B is a side view of the stent of Figure 4A.
Figure 5A is a top view of a fluted stent in accordance with the present invention showing an offset pattern of large grooves and small grooves.
Figure 5B is a side view of the stent of Figure 5A.

### Detailed Description of the Preferred Embodiments

This invention deals with an absorbable/biodegradable, radially fluted, tubular polymeric stent having at least two grooves or flutes along its entire length for expansion after deployment through outward deformation of said grooves to yield essentially circular cross-section. The number of the grooves may also vary between 3 and 12, but preferably between 3 and 9, more preferably between 3 and 6. When more than 3 grooves are present, the depth of the grooves can be equal or unequal to allow for modulated outward deformation to one or more level of expansion and hence, one or more final diameter or cross-section. Another aspect of the invention deals with absorbable polymers for producing absorbable/ biodegradable, radially fluted, tubular stents comprising absorbable crystalline polyesters derived from one or more cyclic monomer selected from the group consisting of *l*-lactide, glycolide, p-dioxanone, trimethylene carbonate, and ε-caprolactone. Another aspect of this invention specifically describes the use of absorbable polymers for producing the stent, wherein the said polymers are made of (1) segmented/block copolyesters wherein one of the segments/blocks is amorphous and the second is crystalline; or (2) segmented/block copolyesters wherein one of the segments/ blocks is crystalline and exhibits a melting temperature Tₘ below 110°C and a second crystalline segment/block having a Tₘ between 140°C and 220°C. These copolyesters are made to have a monocentric polyaxial system comprising a low melting core with high Tₘ segments/blocks extending outward.

Another aspect of this invention deals with an absorbable/biodegradable, radially fluted, tubular polymeric stent comprising a blend of at least two absorbable polymers comprised of dispersed phase of highly crystalline microrods in an amorphous or moderately crystalline matrix. Another aspect of this invention deals with an absorbable/biodegradable, radially fluted, tubular stent comprising a chitosan-based material coated with an absorbable polyester, wherein the chitosan-based material is an acylated chitosan. Another aspect of the invention deals with absorbable/biodegradable, radially fluted, tubular polymeric stents produced by injection molding or thermoforming of an extruded tube. Another aspect of this invention deals with an absorbable/biodegradable fluted tubular stent produced by thermoforming of a non-woven or partially non-woven tube produced, in part or fully, by electrostatic spinning of one of more absorbable polymer. Another aspect of the invention deals with an absorbable/ biodegradable, radially fluted, tubular polymeric stent, wherein the outer wall of said unexpanded stent comprises radially extending barbs to restrict movement of the deployed expanded stent. Another aspect of this invention deals with an absorbable/biodegradable, radially fluted, tubular polymeric stent produced by injection molding. A specific aspect of this invention deals with an absorbable/biodegradable, radially fluted, tubular stent having a continuous cell microporous structure formed by electro-spinning of a viscous solution of constituent polymer(s), wherein the viscous solution may comprise chitosan-based materials, the microporous structure may further comprise an absorbable polyester coating, and the chitosan-based material is made of an acylated chitosan. Another specific aspect of this invention deals with an absorbable/ biodegradable, radially fluted, tubular polymeric stent containing one or more bioactive agent for preventing restenosis and infection.

A specific aspect of this invention deals with an absorbable/biodegradable, radially fluted, tubular polymeric stent for use in vascular and urinogenital applications. A specific aspect of this invention deals with an absorbable, expandable, unfluted, tubular, polymeric stent comprising radially extending barbs, and preferably the wall of the unexpanded form is perforated and the stent is made of a segmented/block copolymer having low- and high-melting crystalline segments/blocks which are present preferably in a polyaxial configuration with the low Tₘ components at the core and the high Tₘ ones extending outward. Another specific aspect of this invention deals with an absorbable/biodegradable, radially fluted, polymeric stent containing at least 10 percent by weight of an inorganic radiopacifier.

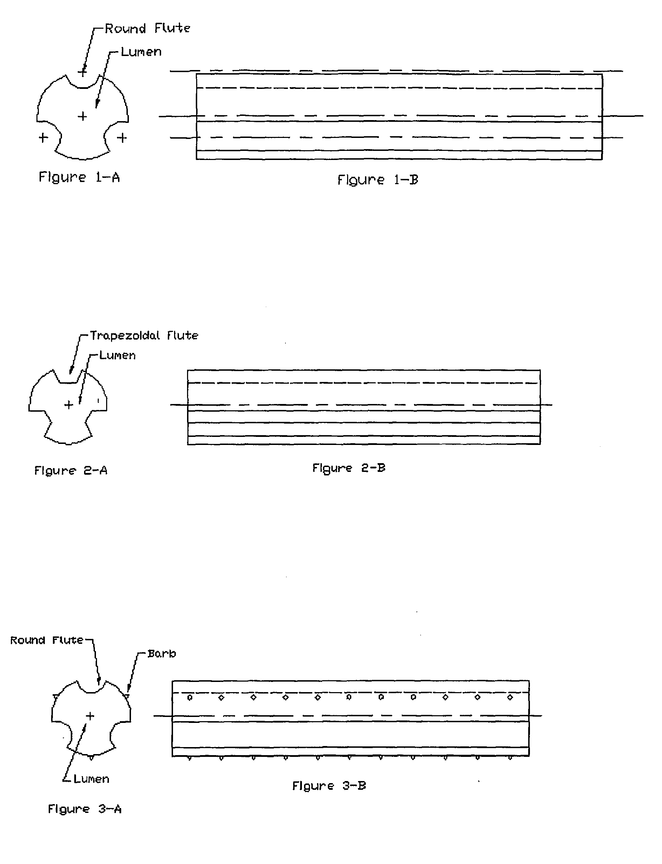

This invention generally deals with absorbable/biodegradable, expandable stents in the form of a radially fluted or perforated tubular configuration. A specific aspect of this invention deals with an absorbable/biodegradable, radially fluted, tubular polymeric stent having at least two and preferably 3-5 and more preferably 6-9 grooves (flutes) along its entire length for expansion after deployment through outward deformation of said grooves to yield essentially a circular cross-section as illustrated in Figures 1-A to 5-B.

Specifically, Figure 1A is a top view of a fluted stent 10 showing the location of three round grooves 12 defined in the stent wall 14. Figure 1B is a side view of the stent of Figure 1A showing that the grooves 12 run the length of the stent 10.

Figure 2A is a top view of a fluted stent 20 with trapezoidal grooves 22 defined in the stent wall 24. Figure 2B is a side view of the stent of Figure 2A.

Figure 3A is a top view of a fluted stent 30 in accordance present invention having grooves 32 defined in the stent wall 34 and barbs 36 extending outwardly from the stent wall 34. Figure 3B is a side view of the stent of Figure 3A.

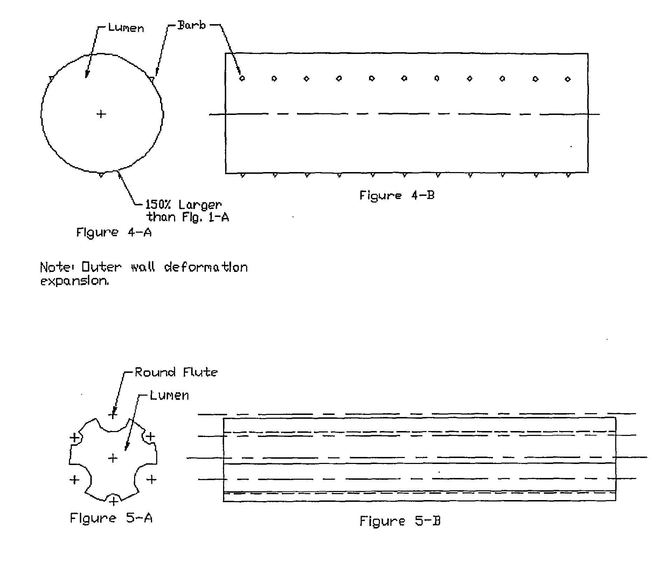

Figure 4A is a top view of an unfluted stent 40 in accordance with the present invention showing the location of the barbs 46 about the stent wall 44. Figure 4B is a side view of the stent of Figure 4A. Such unfluted embodiment will be larger in cross-section than the unexpanded fluted stents of the present invention.

Figure 5A is a top view of a fluted stent 50 in accordance with the present invention showing an offset pattern of large grooves 52 and small grooves 52' defined in the stent wall 54. Figure 5B is a side view of the stent of Figure 5A.

The number and dimension of the flutes can be varied to provide a range of predetermined diameter or cross-sectional area of the expanded stent. Accordingly, a specific aspect of this invention deals with an absorbable stent for temporary placement in body lumens suitable for expansion to a predetermined range of diameters or cross-sections that can be further modulated periprocedure or during deployment of said stent, using a balloon catheter, to achieve more than one level of expansions that are attainable through variability in the shape and depth of the flutes or grooves as exemplified by the two series of flutes per stent depicted in Figs. 5-A and 5-B. Another specific aspect of this invention addresses the composition of the radially fluted, absorbable/biodegradable stent, as being a crystalline polyester derived from one or more cyclic monomer selected from the group consisting of /lactide, glycolide, p-dioxanone, trimethylene carbonate, a morpholine-2,5-dione (substituted or unsubstituted), and ε-caprolactone. Another specific aspect of this invention pertains to the chain sequence distribution of the absorbable/ biodegradable polymer used to prepare said stent wherein the polymeric chains are segmented/block copolyesters wherein one segment/block is crystalline and melts below 110°C and the second segment/block is crystalline but melts between 140°C and 220°C.

Another specific aspect of this invention deals with an absorbable/biodegradable, radially fluted, tubular stent comprising a blend of at least two absorbable polymers comprising a dispersed phase of highly crystalline microrods/nanorods in an amorphous or moderately crystalline matrix. The interfacial tension between the two phases is controlled so as to prevent total miscibility or macrophase separation. The fraction of microrods/nanorods in the blend is adjusted to a maximum value needed to allow for an intimate physical interaction among the microrods/nanorods to provide stiffness and dimensional stability of the expanded stent.

Another specific aspect of this invention calls for an absorbable/biodegradable, radially fluted, polymeric stent comprising a segmented/block copolyester based on a monocentric polyaxial system comprising a low melting temperature (Tₘ) core with high Tₘ crystalline segment/block extending outward. Having two crystalline components with vastly different Tₘ is intended to facilitate and control the effective functional expansion of the stent, and particularly flutes of variable depth, wherein a fluted configuration is heated to liquefy the low Tₘ component during processing and force it to maintain most of its amorphous fraction during storage under the mechanical strain exerted by the rigid, highly crystalline, high Tₘ component of the polymer. The strained component will then be able to recrystallize under the effect of the shear forces developed upon expansion at body temperature.

A different aspect of this invention deals with an absorbable/biodegradable, radially fluted, tubular stent comprising a chitosan-based material and preferably an acylated chitosan coated with a lubricious coating. The latter is intended to facilitate the stent deployment and prevent the blood component(s) from aggregation on the polycationic chitosan or the weakly cationic surface of less-than-completely acylated chitosan.

Another specific aspect of this invention deals with a radially fluted, tubular, absorbable/ biodegradable stent having radially extending barbs on the outside surface between the radially extending grooves. The barbs can be placed so as to provide adequate contact sites with a blood vessel after deployment and expansion along the length of the stent during angioplasty. The barbs are intended to anchor the expanded stent at the intended site and prevent its movement, or migration, along the lumen of the blood vessel.

Another aspect of this invention pertains to methods of producing the radially fluted, absorbable/biodegradable stent. Thus, for stents made of thermoplastic polymers, they can be produced by (1) thermoforming a cylindrical tube with circular cross-sections (typically made by a process entailing electrostatic spinning, injection molding, and/or extrusion) about the surface of a highly polished or Teflon-coated, metallic receptacle equipped for heating and vacuum application; and (2) injection molding using, for instance, a multiple-part mold. Production of a radially fluted stent of soluble polymers can be accomplished using electrostatic spinning (or simply, electro-spinning) of a viscous solution of said polymer or mixture of polymers onto the surface of a Teflon or Teflon-coated rotating mandrel, contoured as a mirror image of the stent lumen. The radially fluted stent prepared by electro-spinning can be made to have solid or microporous walls, wherein the microporosity is associated with an open cell structure. The microporosity is expected to enhance the mass transport of fluids and oxygen across the stent to eliminate or minimize tissue inflammatory response over time periods following angioplasty and allied clinical procedures. Similarly, the electro-spinning can be used to produce chitosan-based stents. Acylation of the chitosan-based stent using, for instance, acetic anhydride is intended to increase the stent rigidity and mechanical stability upon expansion. Coating the chitosan-based stent with a polyester coating can be done by solution dipping and spraying the lumen as well as the outer surface of the stent. A more specific aspect of this invention deals with a fluted stent with perforated walls to facilitate mass transport of nutrients and oxygen across the stent wall to increase biocompatibility and minimize tissue response as discussed earlier for a microporous stent.

Another key aspect of this invention deals an absorbable, expandable, tubular stent with or without radially extending barbs along the outside wall for proper anchoring of the expanded stent. A specific aspect of this invention deals with a tubular, unfluted stent with perforated walls to facilitate the transport of nutrients and oxygen across the stent walls and improve biocompatibility. Another specific aspect of this invention deals with the methods of producing said tubular, unfluted stent. These entail injection molding as described earlier for the fluted stent. An important aspect of this invention pertaining to the tubular stent is the type of polymers which are most suitable for the unfluted, tubular stent that will allow its expansion into a dimensionally stable form that will resist stress-relaxation which may interfere with its functional performance that requires essentially stable lumen dimensions during the critical healing period of a body lumen, such as a blood vessel, when the stent is used in conjunction with angioplasty. More specifically, the unfluted, tubular stent can be made of a segmented/block copolymer having a low melting and high melting crystallites exhibiting melting temperatures (Tₘ) of less than 110°C and 220°C, respectively and preferably less than 60°C and 210°C, respectively. Using such composition will allow the proper morphology development in the ready-to-deploy stent. This entails expanding the tubular stent into the expected functional dimensions (as, for instance, in the case of a blood vessel) where both crystalline fractions (low and high Tₘ) are fully crystallized. The expanded stent will then be placed on a mandrel with dimensions equivalent to those of the ready-to-deploy stent and allow it to shrink at a temperature slightly above the Tₘ of the low Tₘ crystallites, followed by rapid cooling to prevent the low Tₘ component from recrystallizing and insure the presence of the low Tₘ component in a practically amorphous form. This will result in an expandable stent with only the high Tₘ crystallite remaining practically intact. After deployment and expansion, the previously frozen low Tₘ component will recrystallize through shear-induced crystallization and provide necessary reinforcement to prevent stress-relaxation and hence, in-use dimensional stability. Having the low Tₘ component in an amorphous form prior to deployment, also facilitates the stent expansion. Block/segmented copolymers that meet the composition requirements may comprise a linear triblock copolymer of a high glycolide-based polymer as the high Tₘ component and high ε-caprolactone-based polymer as the low Tₘ component, with the latter being the central block in the chain. A preferred composition will comprise a polyaxial block/segmented copolymer with a high caprolactone-based polymeric core with the high glycolide-based polymeric segment extending outward.

Another key aspect of this invention deals with the use of the fluted and unfluted stents in maintaining patency in any body conduit and particularly as a vascular or urinogenital stent. More specific applications of the stents subject of this invention include their use in the urethra, artery, vein, bilary duct, and esophagus.

Another aspect of this invention is surface coating of the fluted or unfluted stents with an absorbable coating to minimize its friction coefficient and facilitate its deployment.

Another aspect of this invention is the incorporation of bioactive agents in the stent matrix or on the surface of the stent for controlled or immediate release at the implantation site, respectively. The bioactive agents can also be a component of a surface coating to allow their controlled release. These agents can be (1) antimicrobial to prevent and/or treat site infection; and (2) one or more of the of the known agent that inhibit any of the biological events that lead to loss of stent functionality, such as restenosis, and particularly in the case of vascular stents.

Another aspect of this invention is the incorporation of an inorganic radiopacifier, such as barium sulfate, at a loading of at least 10 percent to aid in monitoring the location of the stent radiographically. A key aspect of this invention deals with an absorbable stent for temporary placement in body lumens suitable for expansion to a predetermined range of diameters or cross-sections that can be further modulated periprocedure or during deployment of said stent, using a balloon catheter, to achieve more than one level of expansions that are attainable through variability in the shape and depth of the flutes or grooves as exemplified by the two series of flutes per stent depicted in Figs. 5-A and 5-B.

Additional illustratibns of the present invention are provided in the following examples:

### Example 1: Synthesis and Characterization of Polyaxial Glycolide/ε-caprolactone Segmented/Block Copolymers Exhibiting Two Extreme Melting

### Temperatures-

### General Method

The synthesis entails two steps. **In the first step,** a polyaxial polycaprolactone was prepared using trimethylolpropane as the initiator, at a monomer/initiator ratio of 500:1 to 700:1, depending on the sought molecular weight of the final polymer, in the presence of stannous octanoate as a catalyst, at a monomer/catalyst molar ratio of 20,000:1 to 30,000:1, depending on the final copolymer composition. Polymerization was conducted in a mechanically stirred, stainless steel reactor under a dry nitrogen atmosphere at 180°C for 1.5 to 3 hours or until practically a complete conversion is achieved. This was determined by in-process monitoring of conversion using gel permeation chromatography (GPC). The polymer melt was allowed to cool slightly below 180°C prior to adding a predetermined amount of glycolide in the second step in the preparative scheme. After adding glycolide, the polymerization mixture was stirred at or slightly above 180°C until a homogeneous melt was obtained. The reaction was then continued at that temperature for 5 to 7 hours, depending on the final copolymer composition. During this period, the stirring was stopped as the melt became highly viscous and copolymer solidification started to take place. At the conclusion of the polymerization period, the copolymer was super-cooled with liquid nitrogen and removed from the reactor. The polymer was ground, dried under reduced pressure (about 0.1 mm Hg) at 25°C, and then heated to about 100°C under reduced pressure for 5 to 10 hours or until a constant weight is realized, signaling the removal of residual unreacted monomer. The dried/annealed polymer was characterized by differential scanning calorimetry (DSC) for its thermal properties. These entailed initial melting temperature (Tₘ) and heat of fusion (ΔH_{f}). For copolymers which dissolved in hexafluoroisopropyl alcohol, their inherent viscosity was determined as a measure of the molecular weight..

### Example 2: Preparation and Characterization of Typical Polyaxial Segmented Copolymer of ε-Caprolactone and Glycolide

Using the general method described in Example 1, typical copolymers, Co-P1 and CoP3, were prepared and characterized. Key properties of these copolymers are summarized in Table I.

**Table I. Analytical Data of Co-P1 to Co-P3 Tested as Ground Specimens and Their Respective Prepolymers**

| Properties | Co-P1 | Co-P2 | Co-P3 |
|---|---|---|---|
| • Weight Average Molecular Weight of | 47 | 51 | 56 |
| Polycaprolactone Prepolymer, kDa | | | |
| • Copolymer Composition: Glycolide to | 60:40 | 60:40 | 60:40 |
| Caprolactone Molar Ratio | | | |
| • Copolymer Inherent Viscosity, dL/g | 1.7 | 1.82 | Insoluble |
| • Thermal Properties: | | | |
| Polycaprolactone Polyaxial Prepolymer | | | |
| Tₘ,°C | 58 | 57 | 56 |
| ΔH_{f}, J/g | 70 | 89 | 94 |
| Final Polyaxial Copolymer^{a} | | | |
| Tₘ₁, °C | 42 | 41 | 41 |
| ΔH_{f1}, J/g | 18 | 18 | 19 |
| Tₘ₂,°C | 221 | 222 | 221 |
| ΔH_{f2}, J/g | 82 | 80 | 77 |

| | | | |
|---|---|---|---|
| ^{a} Tₘ₁ and ΔH_{f1} = Tm and ΔH of polycaprolactone block/segment. Tₘ₂ and ΔH_{f2} = Tm and ΔH of polyglycolide block/segment. | | | |

### Example 3: Characterization of Thermally and Mechanically Treated Polyaxial Copolymer,

### Co-P2

Co-P2 was subjected to thermal and mechanical treatments similar to those expected to be encountered in key typical processes of those associated with stent fabrication and deployment at the desired biological site. Thin polymer films (0.2 mm) were compression molded at about 235°C under a dry nitrogen atmosphere to provide test specimens for studying the effects of thermal and mechanical treatment (starting with the annealed, ground polymer and unannealed, unoriented films) on melting temperature (Tₘ) and heat of fusion (ΔH_{f}) of the polycaprolactone and polyglycolide constituent blocks/segments of the polyaxial copolymer. The specimens were used to determine changes in Tₘ and ΔH_{f} as a result of annealing and/or uniaxial orientation in tensile mode. Summary of the experimental data are depicted in Table II.
The sets of experiments noted in Table II were designed to determine the effects of (1) melt-processing conditions on percent crystallinity (in terms of ΔH_{f}) and crystallite imperfection and size, i.e., morphology (in terms of Tₘ) as they relate to the fabrication of the stent by injection molding; and (2) uniaxial orientation on percent crystallinity and crystallite morphology as they relate to shear induced crystallization of the stent upon radial balloon expansion during deployment.

**Table II. Differential Scanning Calorimetry Data of Thermally and Mechanically Treated Films of Co-P1**

| | Caprolactone Block/Segment Data | | Polyglycolide Block/Segment Data | |
|---|---|---|---|---|
| Type/Stage of Treatment | Tₘ₁, °C | ΔH_{f1}, J/g | Tₘ₂, °C | ΔH_{f2}, J/g |
| • Dried, thermally annealed ground polymer | 41 | 18 | 222 | 80 |
| • Compression-molded, quick-cooled 0.2 mm thick, Film F1 | 44 | 5 | 225 | 47 |
| • F1 after standing at 25°C for 24 hours, F2 | 44 | 6 | 225 | 49 |
| • F2 after 100 percent elongation during uniaxial tensile orientation, F3 | 42 | 30 | 224 | 65 |
| • F3 after annealing for 24 hours at 37°C, F4 | 43 | 30 | 224 | 66 |

Although the present invention has been described in connection with the preferred embodiments, it is to be understood that modifications and variations may be utilized without departing from the principles and scope of the invention, as those skilled in the art will readily understand. Moreover, Applicants hereby disclose all subranges of all ranges disclosed herein. These subranges are also useful in carrying out the present invention.

## Claims

1. An absorbable, biodegradable, radially fluted, tubular polymeric stent for temporary placement in body lumens having at least two grooves extending along its entire length for expansion after deployment through outward deformation of said grooves to yield an essentially circular cross-section, **characterized in that** the polymeric stent comprises a segmented/block copolyester wherein one of the segments/blocks is crystalline and exhibits a melting temperature (Tₘ) of less than about 110°C and another crystalline segment/block exhibits a melting temperature (Tₘ) of from about 140°C to about 220°C.

2. An absorbable, biodegradable, radially fluted, tubular polymeric stent as set forth in claim 1 wherein the copolyester comprises a monocentric polyaxial system comprising polyaxial core segments having segments/blocks extending outwardly therefrom, wherein the core segments have a lower melting temperature (Tₘ) than the outwardly extending segments/blocks.

3. An absorbable, biodegradable, radially fluted, tubular polymeric stent as set forth in claim 1 wherein the polymeric stent comprises a blend of at least two absorbable polymers comprising a dispersed phase of crystalline microrods and/or nanorods in a crystalline matrix, wherein the microrods and/or nanorods exhibit a higher degree of crystallinity than the matrix.

4. An absorbable, biodegradable, radially fluted, tubular polymeric stent as set forth in claim 1 wherein the polymeric stent comprises a chitosan-based material coated with an absorbable polyester.

5. An absorbable, biodegradable, radially fluted, tubular polymeric stent as set forth in claim 4 wherein the chitosan-based material is an acylated chitosan, preferably coated with a lubricious coating.

6. An absorbable, biodegradable, radially fluted, tubular polymeric stent as set forth in claim 4 or 5 having a microporous structure.

7. An absorbable, biodegradable, radially fluted, tubular polymeric stent as set forth in any one of claims 1 to 6 comprising an absorbable crystalline polyester comprising chain sequences derived from at least one cyclic monomer selected from the group consisting essentially of *l*-lactide, glycolide, p-dioxanone, trimethylene carbonate, ε-caprolactone, and a morpholine-2,5-dione.

8. An absorbable, biodegradable, radially fluted, tubular polymeric stent as set forth in any one of claims 1 to 7 having from 3 to 12 grooves, preferably from 3 to 5 grooves and more preferred from 6 to 9 grooves extending along the entire length thereof.

9. An absorbable, biodegradable, radially fluted, tubular polymeric stent as set forth in claim 8 having more than 3 grooves, the depth of the grooves being equal or unequal to allow for modulated outward deformation resulting in different final diameter after deployment.

10. An absorbable, biodegradable, radially fluted, tubular polymeric stent as set forth in any one of claims 1 to 9 wherein the wall of the stent is perforated.

11. An absorbable, biodegradable, radially fluted, tubular polymeric stent as set forth in any one of claims 1 to 10 wherein the outer wall of said unexpanded stent comprises radially extending barbs between the radially extending grooves to restrict movement of the deployed expanded stent.

12. An absorbable, biodegradable, radially fluted, tubular polymeric stent as set forth in any one of claims 1 to 11 containing at least one bioactive agent for preventing restenosis and infection.

13. An absorbable, biodegradable, radially fluted, polymeric stent as set forth in any one of claims 1 to 12 containing at least 10 percent by weight of an inorganic radiopacifier.

14. An absorbable, biodegradable, radially fluted, polymeric stent as set forth in any one of claims 1 to 13 being a vascular stent.

15. An absorbable, biodegradable, radially fluted, polymeric stent as set forth in any one of claims 1 to 13 being a ureteral stent for the genitourinary tract.

16. Process of making an absorbable, biodegradable, radially fluted, tubular polymeric stent as set forth in any one of claims 4 to 15 comprising the steps of forming the tube by electrostatic spinning of a viscous solution of the constituent polymer or polymer blend, thereby providing a tube having a continuous cell microporous structure, thermoforming the grooves therein and coating the formed tube with an absorbable polyester coating.

17. Process as set forth in claim 16 further comprising the step of acylating the formed tube prior to the step of coating with the absorbable polyester.

## Patentansprüche

1. Absorbierbarer, biologisch abbaubarer, radial gerillter, röhrenförmiger polymerer Stent für das vorübergehende Einbringen in Lumen eines Körpers, der zumindest zwei Rillen aufweist, die sich über seine gesamte Länge erstrecken, für eine Aufweitung nach dem Entfalten durch nach außen gerichtete Verformung der genannten Rillen, um einen im wesentlichen kreisförmigen Querschnitt zu erhalten, **dadurch gekennzeichnet, daß** der polymere Stent einen segmentierten/Block-Copolyester aufweist, wobei eines der Segmente/einer der Blöcke kristallin ist und eine Schmelztemperatur (Tₘ) von weniger als etwa 110°C aufweist, und ein weiteres kristallines Segment/ein weiterer kristalliner Block eine Schmelztemperatur (Tₘ) von etwa 140°C bis etwa 220°C aufweist.

2. Absorbierbarer, biologisch abbaubarer, radial gerillter, röhrenförmiger polymerer Stent nach Anspruch 1, wobei der Copolyester ein monozentrisches, polyaxiales System umfaßt, mit polyaxialen Kernsegmenten, die Segmente/Blöcke aufweisen, die sich davon ausgehend nach außen erstrecken, wobei die Kernsegmente eine geringere Schmelztemperatur (Tₘ) aufweisen als die sich nach außen erstreckenden Segmente/Blöcke.

3. Absorbierbarer, biologisch abbaubarer, radial gerillter, röhrenförmiger polymerer Stent nach Anspruch 1, wobei der polymere Stent eine Mischung von zumindest zwei absorbierbaren Polymeren aufweist, die eine disperse Phase von kristallinen Mikro- und/oder Nanostäbchen in einer kristallinen Matrix umfassen, wobei die Mikro- und/oder Nanostäbchen einen höheren Kristallisationsgrad aufweisen als die Matrix.

4. Absorbierbarer, biologisch abbaubarer, radial gerillter, röhrenförmiger polymerer Stent nach Anspruch 1, wobei der polymere Stent ein Material auf der Basis eines Chitosans umfaßt, das mit einem absorbierbaren Polyester beschichtet ist.

5. Absorbierbarer, biologisch abbaubarer, radial gerillter, röhrenförmiger polymerer Stent nach Anspruch 4, wobei das Chitosan-basierte Material ein acyliertes Chitosan ist, vorzugsweise beschichtet mit einem gleitfähigen Überzug.

6. Absorbierbarer, biologisch abbaubarer, radial gerillter, röhrenförmiger polymerer Stent nach Anspruch 4 oder 5, der eine feinporige Struktur aufweist.

7. Absorbierbarer, biologisch abbaubarer, radial gerillter, röhrenförmiger polymerer Stent nach einem der Ansprüche 1 bis 6, der einen absorbierbaren kristallinen Polyester umfaßt, der Kettensequenzen enthält, die von mindestens einem ringförmigen Monomer abgeleitet sind, das ausgewählt ist aus der Gruppe im wesentlichen bestehend aus 1-Lactid, Glykolid, p-Dioxanon, Trimethylencarbonat, Caprolacton und einem Morpholin-2,5-dion.

8. Absorbierbarer, biologisch abbaubarer, radial gerillter, röhrenförmiger polymerer Stent nach einem der Ansprüche 1 bis 7, der 3 bis 12 Rillen, vorzugsweise 3 bis 5 Rillen und besonders bevorzugt 6 bis 9 Rillen aufweist, die sich über seine gesamte Länge erstrecken.

9. Absorbierbarer, biologisch abbaubarer, radial gerillter, röhrenförmiger polymerer Stent nach Anspruch 8, der mehr als 3 Rillen aufweist, wobei die Tiefe der Rillen gleich oder ungleich ist, um eine modulierte Verformung nach außen zu ermöglichen, die nach der Entfaltung zu verschiedenen Enddurchmessern führt.

10. Absorbierbarer, biologisch abbaubarer, radial gerillter, röhrenförmiger polymerer Stent nach einem der Ansprüche 1 bis 9, wobei die Wand des Stents perforiert ist.

11. Absorbierbarer, biologisch abbaubarer, radial gerillter, röhrenförmiger polymerer Stent nach einem der Ansprüche 1 bis 10, wobei die äußere Wandung des genannten, nicht aufgeweiteten Stents radial sich erstreckende Spitzen zwischen den radial sich erstreckenden Rillen umfaßt, um die Bewegung des entfalteten, aufgeweiteten Stents zu begrenzen.

12. Absorbierbarer, biologisch abbaubarer, radial gerillter, röhrenförmiger polymerer Stent nach einem der Ansprüche 1 bis 11, der zumindest ein biologisch wirksames Mittel enthält, um eine erneute Verengung und eine Infektion zu verhindern.

13. Absorbierbarer, biologisch abbaubarer, radial gerillter, röhrenförmiger polymerer Stent nach einem der Ansprüche 1 bis 12, der zumindest 10 Gew.-% eines anorganischen Röntgenkontrastmittels enthält.

14. Absorbierbarer, biologisch abbaubarer, radial gerillter, röhrenförmiger polymerer Stent nach einem der Ansprüche 1 bis 13, in Form eines vaskularen Stents.

15. Absorbierbarer, biologisch abbaubarer, radial gerillter, röhrenförmiger polymerer Stent nach einem der Ansprüche 1 bis 13, in Form eines Harnröhrenstents für den Urogenitaltrakt.

16. Verfahren zur Herstellung eines absorbierbareren, biologisch abbaubaren, radial gerillten, röhrenförmigen polymeren Stents nach einem der Ansprüche 4 bis 15, das die Schritte umfaßt, Ausbilden eines Röhrchen durch elektrostatisches Verspinnen einer viskosen Lösung des Polymerbestandteils oder Polymergemischs, wodurch ein Röhrchen bereitgestellt wird, das eine durchgehende mikroporöse Zellstruktur aufweist, Thermoformen der Rillen darin und Beschichten des geformten Röhrchens mit einer absorbierbaren Polyesterbeschichtung.

17. Verfahren nach Anspruch 16, das des weiteren den Schritt umfaßt, das geformte Röhrchen vor dem Beschichten mit dem absorbierbaren Polyester zu acylieren.

## Revendications

1. Stent polymère, tubulaire, absorbable, biodégradable, à rainure radiale, pour le placement temporaire dans des cavités corporelles, ayant au moins deux rainures qui s'étendent sur toute de sa longueur pour expansion après déploiement par déformation vers l'extérieur de lesdites rainures pour obtenir pour l'essentiel une coupe transversale circulaire, **caractérisé en ce que** le stent polymère comprend un copolyester segmenté/du bloc, dans lequel un des segments/blocs est cristallin et possède une température de fusion (Tₘ) de moins qu'environ 110°C et un autre segment/bloc cristallin possède une température de fusion (Tₘ) d'environ 140°C à environ 220°C.

2. Stent polymère, tubulaire, absorbable, biodégradable, à rainure radiale selon la revendication 1, dans lequel le copolyester comprend un système monocentrique, polyaxial comprenant des segments de l'âme polyaxials ayant des segments/blocs s'étendant par là vers l'extérieur, dans lesquelles les segments de l'âme ont une température de fusion (Tₘ) plus basse que les segments/blocs s'étendant vers l'extérieur.

3. Stent polymère, tubulaire, absorbable, biodégradable, à rainure radiale selon la revendication 1, dans lequel le stent polymère comprend un composé au moins de deux polymères absorbables comprenant une phase de dispersion des microbâtonnets et/ou nanobâtonnets cristallins dans une matrice cristalline, dans laquelle les microbâtonnets et/ou nanobâtonnets ont un degré de cristallinité supérieur que la matrice.

4. Stent polymère, tubulaire, absorbable, biodégradable, à rainure radiale selon la revendication 1, dans lequel le stent polymère comprend un matériau à base de chitosane enduit avec un polyester absorbable.

5. Stent polymère, tubulaire, absorbable, biodégradable, à rainure radiale selon la revendication 4, dans lequel le matériau à base de chitosan est un chitosane acylée, de préférence enduit avec un revêtement glissant.

6. Stent polymère, tubulaire, absorbable, biodégradable, à rainure radiale selon les revendications 4 ou 5, ayant une structure microporeuse.

7. Stent polymère, tubulaire, absorbable, biodégradable, à rainure radiale, selon une des revendications 1 à 6, comprenant un polyester absorbable, cristallin comprenant séquences de chaîne dérivées au moins d'un monomère cyclique sélectionné parmi le groupe consistant essentiellement de 1-lactide, glycolide, p-dioxanone, trimethylene carbonate, ε-caprolactone, et un morpholine-2,5-dione.

8. Stent polymère, tubulaire, absorbable, biodégradable, à rainure radiale selon une des revendications 1 à 7, ayant de 3 à 12 rainures, de préférence de 3 à 5 rainures et plus préféré de 6 à 9 rainures, s'étendant sur toute de sa longueur.

9. Stent polymère, tubulaire, absorbable, biodégradable, à rainure radiale selon la revendication 8, ayant plus de 3 rainures, la profondeur des rainures étant pareille ou différente pour permettre un déformation modulé par là vers l'extérieur résultant dans un diamètre final différent après le déploiement.

10. Stent polymère, tubulaire, absorbable, biodégradable, à rainure radiale selon une des revendications 1 à 9, dans lequel la face du stent est perforée.

11. Stent polymère, tubulaire, absorbable, biodégradable, à rainure radiale selon une des revendications 1 à 10, dans lequel la face extérieure de ledit stent non élargi comprend des pointes s'étendent radiales entre les rainures s'étendent radiales pour limiter le mouvement du stent déplié et élargi.

12. Stent polymère, tubulaire, absorbable, biodégradable, à rainure radiale selon une des revendications 1 à 11, comprenant au moins un agent bioactive pour éviter un rétrécissement de nouveau et une infection.

13. Stent polymère, tubulaire, absorbable, biodégradable, à rainure radiale selon une des revendications 1 à 12, comprenant au moins 10 pour cent en poids d'un agent radio-opaque anorganique.

14. Stent polymère, tubulaire, absorbable, biodégradable, à rainure radiale selon une des revendications 1 à 13, étant un stent vasculaire.

15. Stent polymère, tubulaire, absorbable, biodégradable, à rainure radiale selon une des revendications 1 à 13, étant un stent urétral pour le système urogénital.

16. Procédé de préparation d'un stent polymère, tubulaire, absorbable, biodégradable, à rainure radiale selon une des revendications 4 à 15, comprenant les étapes de constituant le tube par filage electrostatique à partir d'une solution visqueuse du composant polymère or composé polymère, en préparant un tube ayant une structure des cellules de microporosité continue, en formant les rainures par la procédé de thermoformage, et enduisant le tube formé avec un revêtement d'un polyester absorbable.

17. Procédé selon la revendication 16, en plus comprenant l'étape d' acylation le tube formé avant l'étape d'enduire avec le polyester absorbable.
